# EUROPEAN PATENT SPECIFICATION

(11) **EP 3 231 383 B1**
(45) Date of publication and mention of the grant of the patent: **06.11.2019**
(21) Application number: 17166203.4
(22) Date of filing: 12.04.2017
(51) Int. Cl.: A61B 18/08

(54) **MEDICAL DEVICE**
MEDIZINISCHE VORRICHTUNG
DISPOSITIF MÉDICAL

(30) Priority: 12.04.2016 US 201662321477 P
(43) Date of publication of application: 18.10.2017
(73) Proprietor: Cook Medical Technologies LLC, Bloomington, IN 47404 (US)
(72) Inventor: PAAMAND, Rune Tore, 2700 Broenshoej (DK); TORP, Allan, 4632 Bjaeverskov (DK); BUTZBACKER, Raimo Urban, 4690 Haslev (DK)
(74) Representative: Garratt, Peter Douglas

(56) References cited:
- EP-A1- 2 853 213
- EP-A2- 2 662 041

## Description

### FIELD

The present disclosure relates generally to medical devices. More specifically, the disclosure relates to a device for occluding or closing a body vessel using a current to heat and/or ablate the body vessel.

### BACKGROUND

There are numerous medical conditions when it is desired or necessary to close a body vessel, including the treatment of aneurysms, arteriovenous malformations, arteriovenous fistulas, for starving organs of oxygen and nutrients, in the treatment or containment of cancerous growths, and so on.

Several techniques are known and in use for closing or occluding such body vessels. Traditionally, vessels have been closed by means of external ligation, which generally must be carried out by an open surgery procedure, with its associated risks, inconvenience, and long patient recovery times. Other, more recent, methods aim to use an endoluminal procedure to insert into the vessel or organ one or more occlusion devices, such as a metal framed occluder, coils, pellets or the like, able to obstruct the flow of blood in the vessel.

In some cases, known devices are less suitable for certain types of endoluminal ablation procedures. For example, many proposed devices involve providing a catheter around which an electrically-conductive element is positioned. Such devices may be of too large a diameter to infiltrate vessels that require treatment, particularly small arteries.

It has been a challenge to develop a small-diameter, flexible device which is robust enough to support an electrically-conductive element for an endoluminal ablation device.

Reference is directed to EP 2 853 213 which discloses a device for enabling the length and/or diameter of the heating segment of a medical treatment device to be adjusted on the fly during a treatment procedure, without a need to interrupt the procedure, thus enabling a single catheter to be used at different locations in a hollow anatomical structure.

Reference is further directed to EP 2 662 041 which discloses a device for treating a hollow anatomical structure (HAS), the device comprising a catheter shaft; a balloon attached at one end of the catheter shaft; and an expandable resistive element on the balloon, wherein the balloon is expandable to place the resistive element in apposition with a wall of the HAS.

### BRIEF SUMMARY

This disclosure provides a medical device and methods for conducting vessel ablation and occlusion. The invention is defined in the appended independent claim, preferred embodiments are described in the dependent claims.

In one embodiment, a device for resistive heating of a body vessel in a body is provided. The device includes a support comprising a proximal end and extending to a distal end, the support defining a longitudinal axis. The device includes a coil helically disposed about the support and being electrically conductive, the coil having a first end and extending to a second end disposed at the distal end. The coil includes an electrical connection point disposed between the first end and the second end to define a first portion of the coil between the first end and the electrical connection point, and a second portion of the coil between the electrical connection point and the second end. The first portion includes a first plurality of first windings and the second portion includes a second plurality of second windings. The first windings having a first average pitch and the second windings having a second average pitch, the second average pitch being less than the first average pitch. The device also incorporates a first insulated wire and a second insulated wire, each of the insulated wires being for connection to a source of electrical current. The first insulated wire is connected to the coil at the electrical connection point. The second insulated wire is connected to the second end. When the insulated wires are connected to the source of electrical current, the second portion allows for resistive heating in the body vessel.

In another configuration of the disclosure, a device for resistive heating of a body vessel in a body is provided. The device includes a support comprising a proximal end and extending to a distal end, the support defining a longitudinal axis. The device also includes a coil which includes an insulated wire helically disposed about the support and extending from the proximal end to the distal end to define a plurality of windings. The insulated wire includes an electrically conductive element having a first connector and a second connector. The first and second connectors are for connection to an electrical source and are disposed at the proximal end. When the device is connected to a source of electrical current, the coil allows for resistive heating of a body vessel.

Various additional features and embodiments will become apparent with the following description. The present disclosure may be better understood by referencing the accompanying figures.

### BRIEF DESCRIPTION OF THE DRAWINGS

FIG. 1 depicts a side view of an endoluminal ablation device in accordance with the principles of the present disclosure;
FIG. 2 depicts a cross-sectional view of the portion of the coil identified in FIG. 1;
FIG. 3 depicts a side view of another example of an endoluminal ablation device in accordance with the principles of the present disclosure;
FIG. 4 depicts a side view of another example of an endoluminal ablation device;
FIG. 5 depicts a side view of another example of an endoluminal ablation device;
FIG. 6 depicts a side view of another example of an endoluminal ablation device;
FIG. 7 depicts a side view of another example of an endoluminal ablation device; and
FIG. 8 depicts steps of a method of use for a device of FIG. 1 or FIGs. 3-7 in accordance with one embodiment of the present disclosure.

### DETAILED DESCRIPTION

The present disclosure will now be described more fully with reference to the accompanying figures, which show various embodiments. The accompanying figures are provided for general understanding of various embodiments and example of method steps. However, this disclosure may be embodied in many different forms. These figures should not be construed as limiting, and they are not necessarily to scale. The following definitions will be used in this application.

"About" or "substantially" mean that two given quantities (e.g. lengths or volumes) are within 10%, preferably within 5%, more preferably within 1%. For example, a first quantity of length can be within 10% of a second length quantity.

"Adjacent" referred to herein is near, near to, or in close proximity with.

"Longitudinally" and derivatives thereof will be understood to mean along the longitudinal axis of the device.

The terms "proximal" and "distal" and derivatives thereof will be understood in the frame of reference of a physician using the device. Thus, proximal refers to locations closer to the physician and distal refers to the locations farther away from the physician (e.g., deeper in the patient's vasculature).

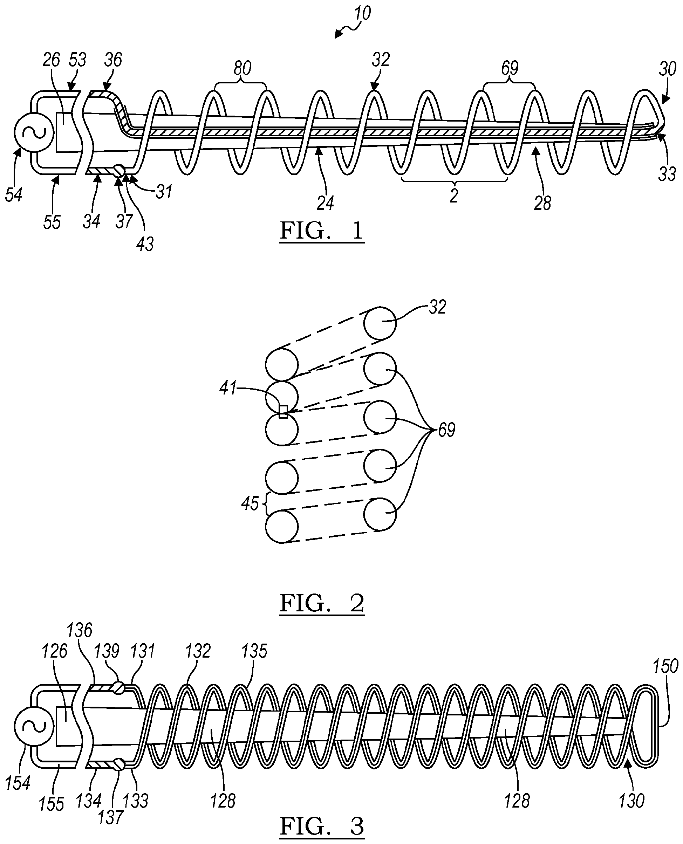

FIG. 1 depicts a side view of one embodiment of a medical device 10 for use in a system that may be used to heat a body vessel at or adjacent a treatment site. The device may have a support 24, or mandrel, that extends from a proximal end 26 to a distal end 30. The support 24 may define a longitudinal axis. The device may have a curvature or be capable of being curved or bent similar to a common wire guide. In some embodiments, the support 24 may be a wire guide. The support 24, having wire guide characteristics, allows for flexibility between the proximal and distal ends of the device 10, which assists in navigation of tortuous vessels.

A resistive heating element, preferably in the form of a coil 32, may be disposed about the support 24, and the coil 32 may be electrically conductive. Although the resistive heating element may take any shape, a helical resistive element such as a coil may be able to produce greater heat within a short length of said element. The electrical conductivity of the coil 32 allows for a current to be passed through a portion of the coil 32, resulting in the coil 32 becoming heated. The coil 32 may have a first end 31 being disposed between the proximal end 26 and distal end 30, and the coil 32 may extend to a second end 33 which is disposed at the distal end 30. The support 24 may have a distal segment 28 that supports the coil 32, or about which the coil 32 is disposed. In some embodiments, the first end 31 of the coil may be mechanically coupled to the support 24. In other embodiments, the first end 31 of the coil 32 may be uncoupled and unattached to the support 24.

The coil 32 may be made up as a plurality of windings 69. Each winding 69 is a single turn of the coil 32 as it proceeds around the support 24. In one embodiment, when the windings are spaced away from the support 24 evenly, a line drawn from a chosen point on one winding such that it is parallel to the longitudinal axis will next intersect the coil on the corresponding point of the next winding.

The resistive element or coil 32 may be uninsulated. Not insulating the resistive element gives better thermal coupling to the treatment site and lower self-heating.

As shown in FIG. 1, the proximal end 26 of the support 24 may be positioned a relatively great distance away from the coil 32. For instance, the proximal end 26 may be close to the operator even when the coil 32 is disposed at or near the site of treatment. The proximal end 26 may be outside of the body during treatment. For example, the support may range from several centimeters to several meters long, while the coil itself only measures about 5 millimeters to about 15 millimeters in length. In some embodiments, the resistive element or coil may be from about 8 millimeters to about 10 millimeters in length. The portion of the support 24 that is surrounded by the coil is referred to as the coil-bearing portion of the support.

The spacing of the windings 69 define a pitch of the coil 32. In an embodiment where the coil 32 takes the shape of a helix, the pitch of the helix is the width as measured of one complete helical turn as measured parallel to its longitudinal axis. A first embodiment as shown in FIG. 1 illustrates a coil with substantially constant pitch 80 along its entire length. However, in other embodiments the pitch will vary in various portions of the coil. In some embodiments, the coil will have different pitch 80 between each of the windings 69.

A cross sectional view of a portion of a coil 32 in accordance with one embodiment of a coil 32 is depicted in FIG. 2. As the coil 32 is placed in a body vessel to be occluded, the windings 69 of the coil may move relative to the support 24 (not shown) or to one another. As shown in FIG. 2, some of the windings 69 may even come in contact with a neighboring winding at a contact point 41. This may cause a short circuit, as electricity will have a shorter path to travel, causing windings that are in contact to only partially heat. However, this may be tolerable in certain situations, and ablation of the vessel may still continue despite this contact. Contact between windings in a single helix configuration will not affect the other windings 69, only those in direct contact. Ordinarily, there will be a space 45 between adjacent windings 69 of the coil, such that electricity flows through the entire length of each winding, allowing each winding to provide thermal energy for heating and ablation of tissue.

The pitch 80 of the coil may, in some portions, be such that the space between the windings 69 of the coil 32 is smaller than the thickness of the windings 69 (meaning the windings are relatively close together, or touching, as shown in FIG. 2). In some cases or portions, the space between the windings 69 may be greater than the thickness of the windings (meaning the windings are generally spaced apart from each other and not touching). This described spacing applies to particular embodiments, but other spacing or lack of spacing between adjacent windings could also be used. The spacing may be consistent or may vary along the length of the device. Greater pitch provides separation between uninsulated adjacent windings of the coil 32 and decreases the chances of a short circuit owing to contact between portions thereof.

In some instances, the pitch may change between each winding of the coil. Therefore it may be more useful to divide the coil into portions and refer to the average pitch of all windings in each of said portions.

The ends 31/33 of the coil may be atraumatic ends. The atraumatic ends may be rounded off in shape, or may be covered, for instance with a shrink tubing. Such atraumatic ends may increase safety as the device is used.

As mentioned, the distal segment 28 may have an outer diameter that distally decreases to form a distal taper (as shown in FIG. 1). Alternatively, the support 24, including its distal segment 28, may have an outer diameter being uniform from the proximal end 26 to the distal end 30. The distal taper or tapered tip is effective to minimize the overall diameter of the device and may make the distal end 30 more flexible and easier to track within the body vessel.

The device 10 may further have a first insulated wire 34 (used interchangeably with the term "first wire") and a second insulated wire 36 (or second wire) for transferring electrical current through coil 32. The first wire 34 may be electrically coupled or connected to a power source 54 and may extend to an electrical connection point 43 on the coil 32. In the device 10 illustrated in FIG. 1, the first end 31 of the coil 32 is coincident with electrical connection point 43. The connection is made between a connector 37 of the electrically-conductive portion of the first insulated wire and an electrically-conductive portion of the coil 32 to form an electrical and conductive coupling between the first wire 34 and the coil 32.

The second wire 36 may also be electrically coupled or connected to the power supply 54 and to the second end 33 of the coil 32. The second wire 36 may be attached to the second end 33 such that the connection provides an electrical coupling between the second wire 36 and the coil 32. In this way, the device may form a first circuit along the path created by the power supply 54, the first wire 34, the coil 32, and the second wire 36. To reduce the overall diameter of the device, the second wire 36 may be positioned between the coil 32 and the support 24, in some cases running along the length of the support 24, in some embodiments being secured thereto. The device may form various circuits, which will be discussed further in FIGs. 3-7.

In general, the first and second insulated wires 34 and 36 will be standard electrical wires, comprising an insulating outer jacket or outer layer with a high dielectric constant that prohibits transmission of electricity therethrough under normal operating conditions, such as an enameled varnish, shrink tubing, molding, or a polymer layer. An inner conductive wire which is surrounded by the outer jacket may be made of a highly conductive material, including but not limited to copper, silver, and platinum. The first and second insulated wires 34/36 may be connected to first lead 53 and second lead 55, respectively, of the power source 54, by any suitable connection. Some examples of connection can include, but are not limited to, soldering, laser welding, press fitting, and so forth. In some embodiments, the first and second insulated wires may be formed integrally with the first and second leads.

The device 10 may optionally have shrink tubing or some other insulative means of confinement disposed about portions thereof. An exemplary shrink tubing may extend around the support 24, the first wire 34, the second wire 36, and/or any other portion of the device 10. The shrink tubing may immobilize or bind the support 24, the first wire 34, and the second wire 36 in place so that they are immobilized relative to each other. The shrink tubing may optionally immobilize and/or extend over a part of the coil 32 to keep the coil 32 in position as the device 10 is in use. A shrink tubing or insulator may in some embodiments be disposed about the support 24, in which case it may act to isolate or insulate the support 24 from the rest of the device 10.

When in use, the system is preferably arranged to operate resistive heating and ablation, using the coil 32 to create a closed loop or circuit. The device may give rise to blood clotting, that is to ablate the blood surrounding the electrical element, and/or to inflame the body vessel to close the vessel. This can be achieved by selecting an ablation energy level and/or an ablation time duration suitable to heat surrounding blood or tissue, which in some circumstances can be expected to include ablation of the vessel tunica, whereby there may be experienced some contraction of the vessel as a result of the heating. The skilled person will be able to determine suitable ablation parameters from common general knowledge in the art. It is to be appreciated that the level of power applied through the resistive element or coil 32 and the time of application will be dependent upon factors including the size of the vessel, the amount of blood flow through the vessel, pulsation and turbulence of blood at the point of ablation, and so on.

In one embodiment, the coil 32 is made from a material having resistive properties, such that heat is generated by electrical current running through it. The coil 32 may further be made from a metal material with thermistor properties. For instance, the resistive element or coil 32 may be made of a nickel alloy. Such alloys include but are not limited to nickel chromium alloys, nickel iron alloys, and nickel magnesium alloys. In a particular embodiment, the coil 32 may be made of a Nifethal material, which is a type of nickel/iron alloy. Particular types of nifethal including Nifethal 70, which is an alloy which is about 72% nickel and a balance of iron. Another nifethal includes Nifethal 52, which is about 52% nickel and the balance being iron.

By using a coil 32 with thermistor properties, the resistance to current delivered to the coil 32 can be used as a measure of temperature of the coil 32. The temperature of the coil is dependent on multiple factors. One factor affecting the temperature of the coil 32 is the current passing through the coil 32. As is typical in resistive heating, as current passes through the coil 32, the coil 32 will become heated. Another factor of the temperature of the coil is the environment in which the coil 32 is disposed. In the case of the present system, the coil 32 is disposed within the body and within a body vessel where blood is flowing, such as through the body vessel. As blood is pumped through the body vessel, the rate of the blood flow will operate to cool the coil 32 as the blood flows past the coil 32. In larger blood vessels, blood flow may be higher than in smaller blood vessels. For example, in an artery with a wider lumen, blood flow is less restricted than in an artery with a narrower lumen. Thus, the temperature of the coil 32 will additionally be affected by the rate of blood flow through the vessel.

During a resistive ablation procedure or occlusion of a blood vessel, the flow rate of the blood flowing through the vessel will decrease as the blood vessel becomes constricted. As the blood flow decreases, the cooling aspects of the flowing blood will also decrease, leading to a resultant increase in the temperature of the coil.

The power supply 54 may be any suitable source of electrical current such that the operating parameters of the device 10 can be met. In one embodiment, the power source may be a source of alternating current (AC) which has a frequency of at least about 100 kilohertz. This frequency threshold allows for effective removal of a direct current component through the coil, which is desirable as direct current may, if not prevented by an insulator, stimulate nerves. The power supply 54 may in some embodiments be a battery and may be coupled to an inverter for the generation of alternating current.

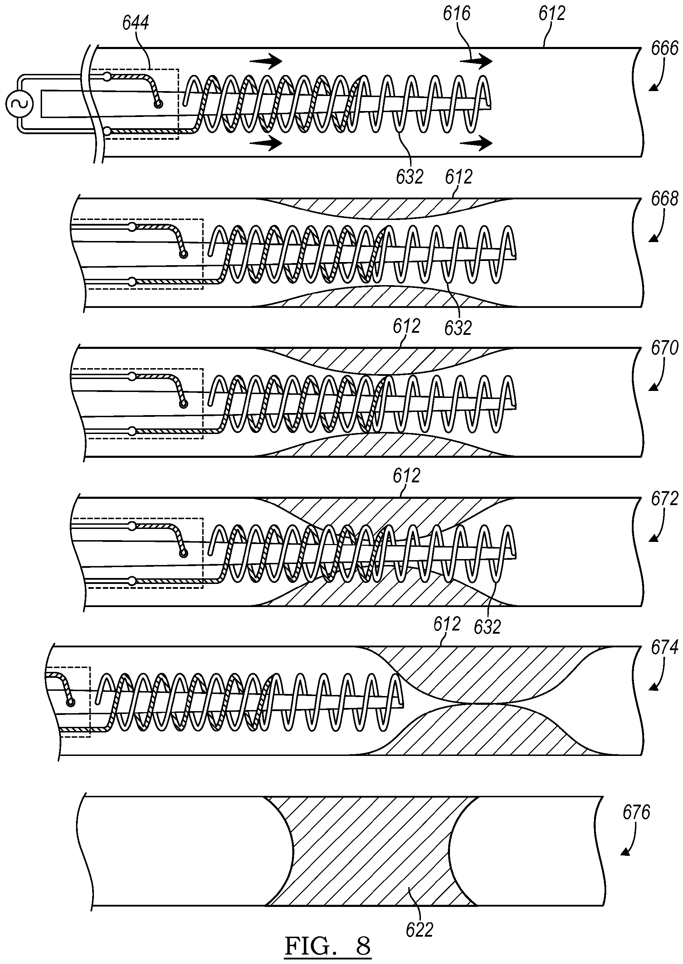

The elements of the system 10 may create various circuits to create an electrical pathway for use in resistive heating. Specifically, the power supply 54 provides current through the first wire 34 the coil 32 via electrical connection point 43. The second wire 36 may also be electrically coupled to the power supply 54 and extend to the second end 33 of the coil 32. The second wire 36 may be attached to the second end 33 of the coil 32. The power supply 54, the first wire 34, the portion of the coil 32 between the electrical connection point 43 and the second end 33, and the second wire 36 form the circuit operable to allow for resistive heating. The system may optionally include an outer sheath (such as sheath 644 shown in FIG. 8) for delivery and/or retrieval of the device into and out of the body. The outer sheath 44 may optionally have a first sheath end (650) and extend to a second sheath end 651. The outer sheath 644 may form an inner lumen 653 therethrough from the first sheath end 650 to the second sheath end 651. The support 624 may be slidably disposed or received within the inner lumen 653.

In one embodiment, the power supply 54 may provide a voltage of about 20 volts to about 50 volts. In such a case, when current is run at about 1 ampere, the wattage is about 20 watts to about 50 watts. In this case the resistivity of the resistive element is about 20 ohm to about 50 ohm. In another embodiment, the current may reach about 2 amperes. In another embodiment, the current may reach about 2.5 amperes, and may be pulsed for about 30% of the active time of the device. In one embodiment, the resistive coil may reach a temperature of about 200 degrees Celsius in about 2 seconds or less from the time that current begins to flow, although in other embodiments the temperature may be lower (such as for example about 90 degrees Celsius.)

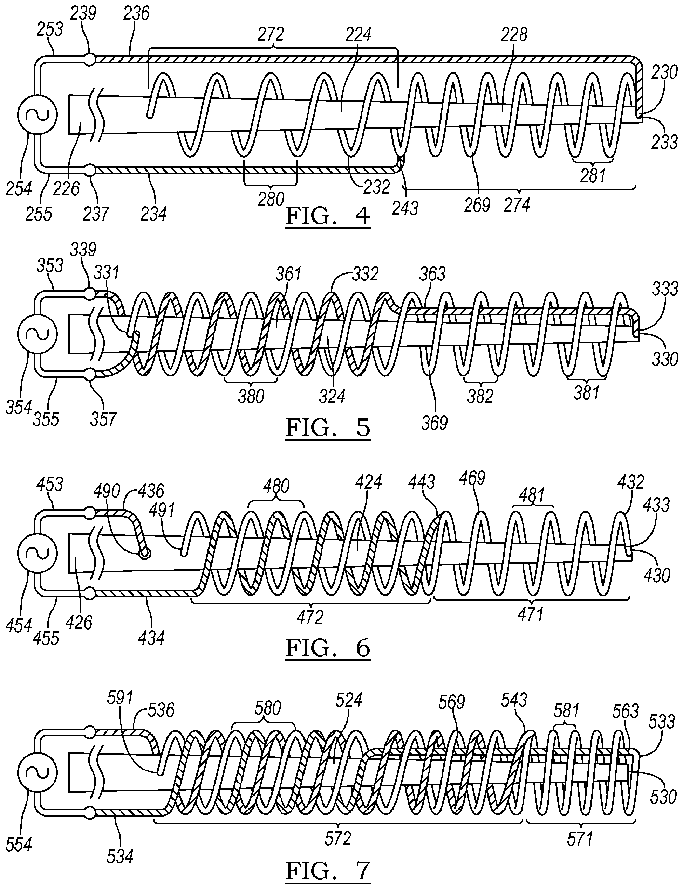

FIGs. 3-7 show further embodiments devices in accordance with the principles of the present disclosure. It will be noted that reference numbers have been kept consistent from one embodiment to the next, and that properties of these portions of the device described previously may also apply to later embodiments. For instance, the materials of coil 32 of FIG. 1 may also be applied to, for example, coil 232 of FIG. 4, or coil 532 of FIG. 7.

FIG. 3 shows another embodiment of a resistive heating device according to the principles of the present disclosure. In this embodiment, the device 110 comprises a double helix-shaped coil 132 which is insulated by insulation 135. Insulation 135 prevents potential contact between points of the double helical coil 132 from shorting. Double helical coil 132 allows for a closed-loop circuit, which enables the patient to avoid being exposed to electrical current.

The coil 132 is a single insulated element or insulated wire with two connectors, first connector 137 and second connector 139, positioned proximally, for connection to first and second insulated wires 134/136. Such proximal connectors allow for minimization of the diameter of the device, as the welds for connection between the insulated coil 135 and the first and second wires 134/136 are kept proximal of the end which is likely to enter the narrowest portions of the vasculature. The coil 132 may terminate in a loop 150 at the portion of the coil 132 closest the distal end 130. In one embodiment, the double helical coil 132 may be laser cut from a cylindrical metal cannula, and covered with an insulating layer 135 thereafter.

The first and second insulated wires 134/136 are in turn connected to leads 153/155, which connect to the power supply 154. In certain embodiments, the first and second insulated wires 134/136 may be integral with their respective leads, giving the appearance of a single contiguous piece of wire apiece.

The insulation 135 may be any material which allows for transmission of thermal energy therethrough, such that ablation of the target vessel can be achieved. The material of the insulation 135 should be capable of tolerating the flow of electrical current through the coil 132 and the heat generated thereby.

In one embodiment, the insulation 135 is a coating of a polymer. In some embodiments, the insulation may be PTFE or PEBAX. In a specific embodiment, the polymer is parylene C. Parylene C is a dielectric coating which can be coated onto materials by a variety of methods, such as vapor deposition, and can be done in very thin layers while still maintaining a pinhole-free layer. In conjunction with a tip of the present disclosure, the layer of parylene C may range from being about 1.5 nanometers thick to about 1 micron thick. In a particular embodiment, the layer of parylene C may be about 0.01 to about 0.5 micron thick, or about 0.05 micron thick to about 0.25 micron thick, or about 0.1 micron thick to about 0.2 micron thick, or about 0.1 micron thick, or about 0.15 micron thick. In the case of a 0.5 mm diameter conductive element, a 0.1 micron coating will produce an impedance of about 70 ohm at the frequency of greater than about 100 kilohertz, such as about 500 kilohertz.

In one embodiment, the coil may be manufactured by laser cutting a cannula. In such an embodiment the coil may be made from nitinol or other suitable materials common for laser cutting medical structures.

FIG. 4 shows another device in accordance with the principles of the present disclosure. In this embodiment, the electrical connection point 243 is located on coil 232 at a position between first end 231 and second end 232. First wire 234 is connected to the coil 232 at the electrical connection point 243, and second wire 236 is connected to the coil 232 at second end 233. The electrical connection point 243 is located on a helical portion of the coil 232, along a length of the helical portion of the coil 243. Because the coil 232 has a higher resistivity than the first insulated wire 234 and the second insulated wire 236, the consequence of this connection arrangement is that the coil 232 becomes effectively divided into two portions: a first portion 272 which is not part of an electrical circuit when current is provided by the power supply 254, and a second portion 274 which is part of the electrical circuit when current is provided by the power supply 254. As a result, the second portion 274 of the coil 232 is the heating element of the device 210.

Materials from which the resistive element may be made that provide the desired resistive properties may have physical properties that make a coil shape, which imparts spring-like tendencies, difficult to handle. Moreover, mere attachment of the coil 232 by its end 233 to the support 224 creates stress that may cause strain to a relatively brittle material. For this reason, the non-electrified first portion 272 of the coil 232 is provided. The first average pitch 280 of the first portion 272 is greater than the second average pitch 281 of the second portion 274, which creates less-dense windings in order to relieve mechanical stress. Such an unstressed tail portion reduces the chances that the coil 232 will kink during deployment, and relieves the stress of the relatively tight, close windings 269 of the second portion 274 of coil 232, and the stress of connecting second end 233 to the support 224. Therefore a feature which may improve the construction of the device may include such a thermally inactive mechanical coupling zone like first portion 272.

FIG. 5 is another embodiment of a device of the present disclosure. The average pitch 380 of the coil 332 is greater toward the proximal end 326 of the support 324 for the reasons disclosed previously. The average pitch 381 of the coil 332 at the second portion 374 is lower, thus the windings 369 are packed more densely together. The first wire 334 connects to the first end 331 of the coil 332, and the second insulated wire 336 winds along the support between windings 369 of the coil 332 for a portion, and then runs through a space between the coil 332 and the support 324 to connect to the second end 333 of the coil 332 in order to minimize the diameter of the device. The entire coil 332 is therefore electrified during operation, and the proximal portion of the coil may provide heat, albeit more dispersely than the distal portion of the coil. Therefore the more proximal portion may serve to act as a preheating element, delivering a lower amount of heat than the dense coil-packing of the distal end. In one mode of operation the heat generated at the proximal portion of the coil is significantly lower that the distal portion of the coil and will not cause tissue damage and ablation.

FIG. 6 is an illustration of a device 410 in accordance with another embodiment of the present disclosure. Here, the connection between the second wire 436 and the second end 433 of the coil 432 is indirect, with the second insulated wire 436 being electrically connected to the support 424 at connection point 490. In such an arrangement, the support 424 is part of the circuit, and in some embodiments the support 424 is covered by an outer insulating layer. The first insulated wire 434 is connected to the coil 432 at electrical connection point 443. Electricity may flow from the power supply 454 through lead 453 to second wire 436, into support 424, through the second portion 471 of the coil, and into first wire 434 via electrical connection point 443, completing the circuit through lead 455 into power supply 454. As shown, the average of pitch 481 of the coil 432 is less than the average of pitch 482 in the first portion 472 of the coil 432, as the windings 469 are more densely packed in second portion 471 than in first portion 472. First insulated wire 434 winds about the support 424 between windings 469 of coil 432 until it reaches electrical connection point 443, which minimizes the overall diameter of the device.

FIG. 7 is another illustration of a device of the present disclosure. The average pitch 580 of the coil 532 is greater toward the proximal end 526 of the support 524 for the reasons disclosed previously. The average pitch 581 of the coil 532 at the second portion 571 is lower, thus the windings 569 are packed more densely together. The first insulated wire 536 connects to the electrical connection point 543 which is between the first end 591 and the second end 533 of the coil 532, and the second insulated wire 536 winds along the support between windings 569 of the coil 532 for a portion, and then runs through a space between the coil 532 and the support 524 to connect to the second end 533 of the coil 532 in order to minimize the diameter of the device. The first and second insulated wires 536/534 are wound around the support 524 in an arrangement such that windings of the second wire are between the first wire and the coil, and windings of the first wire are between windings of the second wire and the coil, and windings 569 of the coil 532 are between one each of the first wire 536 and second wire 534.

The coil 532 has second portion 571 which is electrified during operation and provides heat during ablation, and second portion 572 proximal/upstream of the first portion 571 which is not electrified and provides mechanical stress relief to the remainder of the coil 532.

FIG. 8 depicts an example of a to fully occlude a body vessel 612. In step 666, the device may be positioned in the body vessel 612 adjacent the treatment site. At this time, blood flow 616 will be substantially normal and flowing at a first rate. In step 668, the system may generate power from the power supply 654, causing current to flow through the first circuit into the coil 632 and heat the electrically-active portion of coil 632.

When the current is flowing, the method may include heating a local zone of the body vessel 612 after the step of generating the power. This step of heating may cause swelling of the body vessel 612 at the treatment site, as depicted in step 668. As shown, the step 668 of first transmitting power may comprise avoiding contact of the coil 632 with the vessel wall 612. However, it is also possible that the coil 632 contact the vessel wall 620 to provide direct contact between the coil 632 and the wall to heat the wall 620 and cause it to swell.

In steps 670 and 672, the device 610 will continue to generate current with the power supply 654, and the vessel wall 612 will further swell. At this point, the step of transmitting a current through the coil may comprise contacting the vessel wall with the coil or contacting additional surface area of the vessel wall.

In step 674, the device 610 may start to fully occlude the body vessel 612. As such, the operator may withdraw the device 610 from the body vessel 12. This withdrawal may be manual or automatic. In step 676, the body vessel has been fully included with occlusion 622. The device may be fully withdrawn.

The operator may detect the occlusion process by assessing the temperature of the device in case the device has thermosensing capabilities (e.g. as previously described by using thermistor materials in the coil). Occlusion may be detected as an increase in temperature without an increase in output power, or in more general terms as a change in the power-to-temperature response, signifying a change in environment or cooling of the device as a result of occlusion.

The above described steps 666-676 provide a general description of resistive heating with the coil 632. Although one embodiment of the device is illustrated in FIG. 8, it is to be understood that a device having any structure disclosed herein, or described herein, can be substituted to achieve a similar end.

It should be understood that the foregoing relates to exemplary embodiments of the disclosure and that modifications may be made without departing from the scope of the disclosure as set forth in the following claims. While the disclosure has been described with respect to certain embodiments it will be appreciated that modifications and changes may be made by those skilled in the art without departing from the scope of the invention as set forth in the appended claims. The present disclosure provides for a device and examples of methods of use to endoluminally ablate and/or occlude a body vessel using a current to perform resistive heating. The device includes a resistive coil, which in some embodiments has a portion with a first, higher average pitch which can be electrified, and a second, lower average pitch, which is not electrified but aids in relieving mechanical stress. The device may have a support, which may be a wire guide, around which the coil is disposed. The device may provide a minimal diameter and flexibility for navigation of tortuous vessels.

## Claims

1. A device for resistive heating of a body vessel in a body, the device comprising:
a support (224) comprising a proximal end and extending to a distal end, the support defining a longitudinal axis;
a coil (232) helically disposed about the support and being electrically conductive, the coil comprising a first end (231) and extending to a second end (232) disposed at the distal end, the coil including an electrical connection point (243) disposed between the first end and the second end to define:
a first portion (272) of the coil between the first end and the electrical connection point, and
a second portion (274) of the coil between the electrical connection point and the second end;
the first portion comprising a first plurality of first windings and the second portion comprising a second plurality of second windings, the first windings having a first average pitch and the second windings having a second average pitch, the second average pitch being less than the first average pitch; and
a first insulated wire (234) and a second insulated wire (236), each of the insulated wires being for connection to a source of electrical current, the first insulated wire being connected to the coil at the electrical connection point, the second insulated wire being connected to the second end, such that when the insulated wires are connected to the source of electrical current, the second portion allows for resistive heating in the body vessel.

2. The device of claim 1, wherein the support is a wire guide.

3. The device of any one of the preceding claims, wherein the support comprises a tapered portion at the distal end.

4. The device of any one of the preceding claims, wherein the coil comprises a thermistor material.

5. The device of any one of the preceding claims, wherein the coil is uninsulated.

6. The device of any one of the preceding claims, wherein the support comprises an outer insulating layer.

7. The device of any one of the preceding claims, wherein a portion of the first or second insulated wire is disposed between the coil and the support.

8. The device of any one of the preceding claims, wherein a portion of the first or second insulated wire is wound about the support between windings of the coil.

9. The device of any one of the preceding claims, wherein the second insulated wire is connected to the support at a point proximal to the distal end, such that a portion of the insulated wires are connected to an electrical source, a portion of the support is electrified, preferably wherein the support comprises an outer insulating layer.

10. The device of any one of the preceding claims, wherein the coil has a resistivity of about 10 ohms to about 100 ohms.

11. The device of any one of the preceding claims comprising a battery.

12. The device of claim 11 comprising an inverter.

## Patentansprüche

1. Vorrichtung zur Widerstandserwärmung eines Körpergefäßes in einem Körper, wobei die Vorrichtung Folgendes umfasst:
ein Stützelement (224), das ein proximales Ende umfasst und sich zu einem distalen Ende erstreckt, wobei das Stützelement eine Längsachse definiert;
eine Spule (232), die spiralförmig um das Stützelement angeordnet und elektrisch leitfähig ist, wobei die Spule ein erstes Ende (231) umfasst und sich zu einem zweiten Ende (232) erstreckt, das an dem distalen Ende angeordnet ist, wobei die Spule einen elektrischen Verbindungspunkt (243) beinhaltet, der zwischen dem ersten Ende und dem zweiten Ende angeordnet ist, um Folgendes zu definieren:
einen ersten Abschnitt (272) der Spule zwischen dem ersten Ende und dem elektrischen Verbindungspunkt, und
einen zweiten Abschnitt (274) der Spule zwischen dem elektrischen Verbindungspunkt und dem zweiten Ende;
wobei der erste Abschnitt eine erste Vielzahl von ersten Wicklungen umfasst und der zweite Abschnitt eine zweite Vielzahl von zweiten Wicklungen umfasst, wobei die ersten Wicklungen eine erste mittlere Ganghöhe aufweisen und die zweiten Wicklungen eine zweite mittlere Ganghöhe aufweisen, wobei die zweite mittlere Ganghöhe geringer als die erste mittlere Ganghöhe ist; und einen ersten isolierten Draht (234) und einen zweiten isolierten Draht (236), wobei jeder der isolierten Drähte mit einer elektrischen Stromquelle verbunden werden soll, der erste isolierte Draht an dem elektrischen Verbindungspunkt mit der Spule verbunden ist, der zweite isolierte Draht mit dem zweiten Ende verbunden ist, sodass, wenn die isolierten Drähte mit der elektrischen Stromquelle verbunden sind, der zweite Abschnitt eine Widerstandserwärmung im Körpergefäß ermöglicht.

2. Vorrichtung nach Anspruch 1, wobei das Stützelement eine Drahtführung ist.

3. Vorrichtung nach einem der vorangegangenen Ansprüche, wobei das Stützelement einen verjüngten Abschnitt am distalen Ende umfasst.

4. Vorrichtung nach einem der vorangegangenen Ansprüche, wobei die Spule ein Thermistormaterial umfasst.

5. Vorrichtung nach einem der vorangegangenen Ansprüche, wobei die Spule nicht isoliert ist.

6. Vorrichtung nach einem der vorangegangenen Ansprüche, wobei das Stützelement eine äußere isolierende Schicht umfasst.

7. Vorrichtung nach einem der vorangegangenen Ansprüche, wobei ein Abschnitt des ersten oder zweiten isolierten Drahts zwischen der Spule und dem Stützelement angeordnet ist.

8. Vorrichtung nach einem der vorangegangenen Ansprüche, wobei ein Abschnitt des ersten oder zweiten isolierten Drahts zwischen Wicklungen der Spule um das Stützelement herum gewickelt ist.

9. Vorrichtung nach einem der vorangegangenen Ansprüche, wobei der zweite isolierte Draht an einem Punkt proximal zu dem distalen Ende mit dem Stützelement verbunden ist, sodass ein Abschnitt der isolierten Drähte mit einer elektrischen Quelle verbunden ist, ein Abschnitt des Stützelements elektrifiziert ist, vorzugsweise wobei das Stützelement eine äußere isolierende Schicht umfasst.

10. Vorrichtung nach einem der vorangegangenen Ansprüche, wobei die Spule einen Widerstand von etwa 10 Ohm bis etwa 100 Ohm aufweist.

11. Vorrichtung nach einem der vorangegangenen Ansprüche, die eine Batterie umfasst.

12. Vorrichtung nach Anspruch 11, die einen Wechselrichter umfasst.

## Revendications

1. Dispositif pour le chauffage résistif d'un vaisseau corporel dans un organisme, le dispositif comprenant :
un support (224) comprenant une extrémité proximale et s'étendant vers une extrémité distale, le support définissant un axe longitudinal ;
une bobine (232) disposée de façon hélicoïdale autour du support et étant électroconductrice, la bobine comprenant une première extrémité (231) et s'étendant vers une seconde extrémité (232) disposée au niveau de l'extrémité distale, la bobine comprenant un point de connexion électrique (243) disposé entre la première extrémité et la seconde extrémité pour définir :
une première partie (272) de la bobine entre la première extrémité et le point de connexion électrique, et
une seconde partie (274) de la bobine entre le point de connexion électrique et la seconde extrémité ;
la première partie comprenant une première pluralité de premiers enroulements et la seconde partie comprenant une seconde pluralité de seconds enroulements, les premiers enroulements possédant un premier pas moyen et les seconds enroulements possédant un second pas moyen, le second pas moyen étant inférieur au premier pas moyen ; et
un premier fil isolé (234) et un second fil isolé (236), chacun des fils isolés étant destiné à une connexion à une source de courant électrique, le premier fil isolé étant connecté à la bobine au niveau du point de connexion électrique, le second fil isolé étant connecté à la seconde extrémité, de sorte que, lorsque les fils isolés sont connectés à la source de courant électrique, la seconde partie permette un chauffage résistif dans le vaisseau corporel.

2. Dispositif selon la revendication 1, dans lequel le support est un guide-fil.

3. Dispositif selon l'une quelconque des revendications précédentes, dans lequel le support comprend une partie conique au niveau de l'extrémité distale.

4. Dispositif selon l'une quelconque des revendications précédentes, dans lequel la bobine comprend un matériau de thermistance.

5. Dispositif selon l'une quelconque des revendications précédentes, dans lequel la bobine n'est pas isolée.

6. Dispositif selon l'une quelconque des revendications précédentes, dans lequel le support comprend une couche isolante externe.

7. Dispositif selon l'une quelconque des revendications précédentes, dans lequel une partie du premier ou du second fil isolé est disposée entre la bobine et le support.

8. Dispositif selon l'une quelconque des revendications précédentes, dans lequel une partie du premier ou du second fil isolé est enroulée autour du support entre les enroulements de la bobine.

9. Dispositif selon l'une quelconque des revendications précédentes, dans lequel le second fil isolé est connecté au support au niveau d'un point proximal par rapport à l'extrémité distale, de sorte qu'une partie des fils isolés soit connectée à une source électrique, qu'une partie du support soit électrifiée, de préférence dans lequel le support comprend une couche isolante externe.

10. Dispositif selon l'une quelconque des revendications précédentes, dans lequel la bobine présente une résistivité d'environ 10 ohms à environ 100 ohms.

11. Dispositif selon l'une quelconque des revendications précédentes comprenant une batterie.

12. Dispositif selon la revendication 11 comprenant un onduleur.
